# EUROPÄISCHE PATENTSCHRIFT

(11) **EP 0 868 171 B1**
(45) Veröffentlichungstag und Bekanntmachung des Hinweises auf die Patenterteilung: **29.01.2003**
(21) Anmeldenummer: 97903343.8
(22) Anmeldetag: 20.02.1997
(51) Int. Cl.: A61K 9/00, A61K 9/20

(54) **IMPLANTATE MIT PHASENWEISER ARZNEISTOFFABGABE**
IMPLANTS WITH PHASED RELEASE OF MEDICAMENTS
IMPLANTS A LIBERATION DE MEDICAMENTS PAR PHASES

(30) Priorität: 05.03.1996 DE 19608423
(43) Veröffentlichungstag der Anmeldung: 07.10.1998
(73) Patentinhaber: Göpferich, Achim Prof. Dr., 93040 Regensburg (DE)
(72) Erfinder: Göpferich, Achim Prof. Dr., 93040 Regensburg (DE)
(74) Vertreter: Ahrens, Gabriele, Dr.
(86) Internationale Anmeldenummer: EP9700820
(87) Internationale Veröffentlichungsnummer: WO97032570

(56) Entgegenhaltungen:
- EP-A- 0 111 144
- WO-A-92/02211
- DE-A- 2 424 169

## Beschreibung

Die Erfindung betrifft neuartige Implantate zur Arzneistoffabgabe. Diese erlauben, denselben Arzneistoff in mehreren Phasen oder mehrere Arzneistoffe in zeitlich vorherbestimmter Reihenfolge freizugeben. Die Implantate bestehen aus Schichten bioabbaubarer Polymere, die innerhalb weniger Wochen im Körper vollständig erodieren.

Die Forschung im Bereich der kontrollierten Arzneistofffreigabe konzentrierte sich ursprünglich darauf, Arzneistoffe mit konstanter Geschwindigkeit freizugeben, um konstante Plasma- bzw. Gewebespiegel zu erzielen. Es wurde jedoch erkannt, daß auch die diskontinuierliche Freigabe von Arzneistoffen sehr vorteilhaft sein kann. Dadurch läßt sich zum Beispiel die Entwicklung von Toleranzen verhindern und bei der Immunisierung lassen sich höhere Immunantworten erzielen. Ein weiteres Anwendungsgebiet für Arzneiformen mit diskontinuierlicher Freigabe ist die Tumortherapie. Tumorzellen neigen zur Entwicklung von Resistenzen, wenn derselbe Wirkstoff kontinuierlich verabreicht wird. Implantate, welche Arzneistoffe diskontinuierlich oder sequentiell freigeben, könnten dieser Entwicklung entgegenwirken. Ein Beispiel für die potentielle Anwendung solcher Implantate ist die Behandlung von Tumoren im Bereich des Zentralen Nervensystems. Es ist daher wünschenswert, lokal implantierbare Arzneistoffabgabesysteme verfügbar zu haben, die eine phasenweise Abgabe von unterschiedlichen Arzneistoffen oder dem selben Arzneistoff in unterschiedlicher Konzentration ermöglichen.

In der deutschen Offenlegungsschrift 2 424 169 sind Implantate zur phasenfreien Freisetzung von Wirkstoffen beschrieben, wobei die Implantate aus Schichten bestehen, die unterschiedliche Arzneistoffe oder denselben Arzneistoff in unterschiedlicher Konzentration enthalten können und die einzelnen Schichten aus denselben oder verschiedenen bioabbaubaren Polyrnermaterialien aufgebaut sind. Hierbei ist in jeder der Schichten ein Gehalt an Wirkstoff vorgesehen.

Gegenstand der Erfindung sind Implantate mit phasenweiser Arzneistoffabgabe, die aus einem Kern und darum konzentrisch angeordneten Schichten bestehen, wobei der Kern und zumindest eine der Schichten unterschiedliche Arzneistoffe oder denselben Arzneistoff in unterschiedlicher Dosis enthalten und die arzneistoffhaltigen Bereiche der Implantate aus oberflächenerodierend bioabbaubaren Polymermaterialien bestehen und zumindest zwischen zwei arzneistoffhaltigen Bereichen mindestens eine arzneistofffreie Schicht aus einem bulkerodierend bioabbaubarem Polymermaterial angeordnet ist.

Durch das Vorsehen von arzneistofffreien Schichten zwischen arzneistoffhaitigen Bereichen kann erfindungsgemäß eine zeitliche Unterbrechung der Wirkstofffreigabe erzielt werden.

Die erfindungsgemäßen Implantate bestehen aus einem Kern und ein oder mehreren konzentrisch darum angeordneten Schichten oberflächenerodierend bioabbaubarer Polymermaterialien, die unterschiedliche Erosionsgeschwindigkeiten besitzen können.

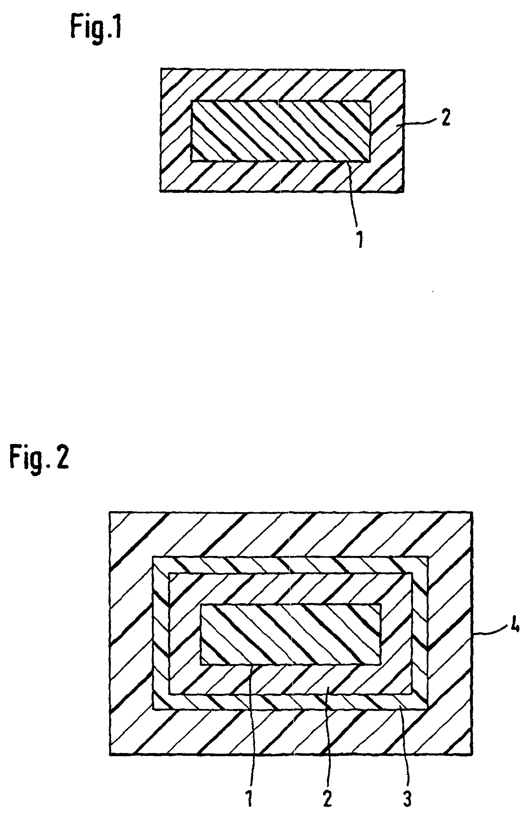

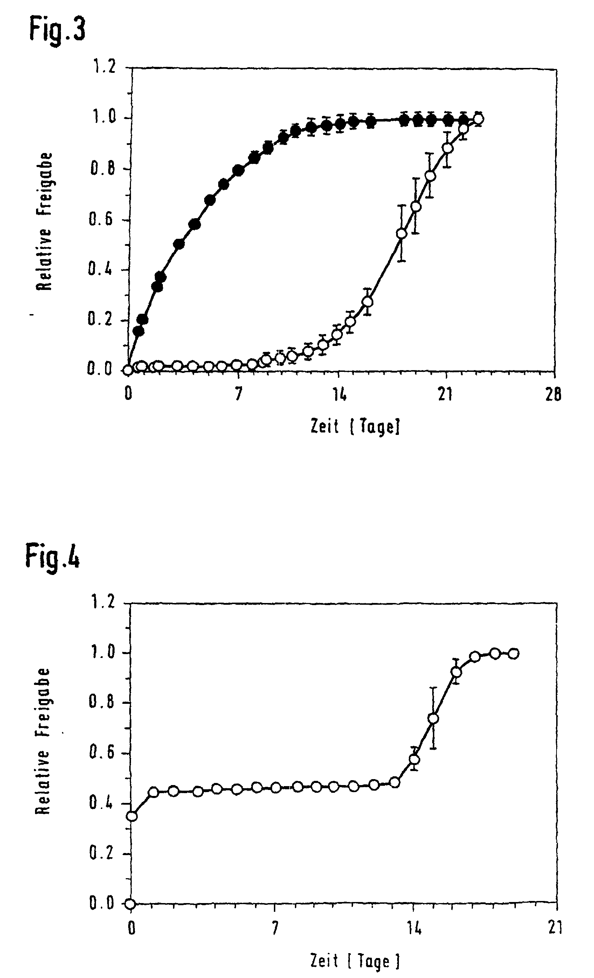

Es zeigen
- Abbildung 1: als Referenzbeispiel den schematischen Aufbau eines zweischichtigen Implantates im Querschnitt. Der Kem 1 ist von einer Hüllschicht 2 umgeben. Kern 1 und Hüllschicht 2 bestehen aus oberflächenerodierend bioabbaubaren Polymermaterialien und enthalten den jeweiligen Arzneistoff.
- Abbildung 2: den schematischen Aufbau eines erfindungsgemäßen vierschichtigen implantates im Querschnitt. Hier wird ein Kern 1 von zwei Zwischenschichten 2 und 3 umgeben. Darum ist eine Mantelschicht 4 angeordnet. Kern 1 und Mantelschicht 4 bestehen aus oberflächenerodierend bioabbaubaren Polymermaterialien und enthalten den jeweiligen Arzneistoff. Die Zwischenschichten 2 und 3 bestehen ebenfalls aus bioabbaubaren Polymermaterialien und sind arzneistofffrei.

In dem Referenzbeispiel entsprechend Abbildung 1 wie es prinzipiell auch auf die vorliegende Erfindung Anwendung finden kann, besteht das Implantat aus einem Arzneistoff-haltigen Kern und einer Arzneistoff-haltigen Hüllschicht. Durch entsprechende Wahl der Polymermaterialien, deren Bioabbaubarkeit im Körper oberflächenerosionskontrolliert ist, ist gewährleistet, daß der jeweilige Arzneistoff entsprechend des Abbaus des Polymermaterials ausschließlich von der Oberfläche her phasenweise abgegeben wird. Die zeitliche Dauer und das Abgabeprofil kann durch Auswahl der Polymermaterialien nach unterschiedlicher Abbaubarkeit geregelt werden. Je nach Dimensionierung von Hüllschicht und kern sowie der Dosierung der darin eingelagerten Arzneistoffe kann deren Abgabe jeweils über Tage oder Wochen hinweg und streng getrennt erfolgen.

Die Arzneistoff-haltigen Bereiche des Implantates können mit unterschiedlichen Arzneistoffen beladen sein. Dies ist sinnvoll, wenn eine Kornbinationstherapie unter zeitlicher Abstufung angestrebt wird. Die Arzneistoff-haltigen Bereiche des Implantates können aber auch ein- und denselben Arzneistoff in unterschiedlicher Dosis enthalten. Hierdurch ist es möglich, durch zeitlich abgestufte, unterschiedliche Arzneistoffdosierung optimiert zu therapieren.

In einer erfindungsgemäßen Ausführungsform, etwa wie in Abbildung 2 gezeigt, sind zwischen den Arzneistoff-haltigen Bereichen 1 und 4 des Implantates arzneimittelfreie Zwischenschichten 2 und 3 angeordnet. Die letzteren bestehen ebenfalls aus bioabbaubaren Polymermaterialien, nämlich aus bulkerodierend abbaubaren Polymermaterialien, die nur langsam abgebaut werden. Hierdurch kann eine noch strengere Trennung der Arzneistoffabgabe aus den verschiedenen Arzneistoff-beladenen Bereichen des Implantates bewirkt werden.

Dem Fachmann sind die vielfältigsten bioabbaubaren Polymermaterialien und deren Abbaueigenschaften bekannt. Eine Auswahl nach oberflächenerodierender oder bulkerodierender Abbaubarkeit ist problemlos, gegebenenfalls durch einfache orientierende Versuche, möglich.

Gegenüber monolithischen Implantaten zeichnen sich die Implantate gemäß der Erfindung durch eine größere Flexibilität in der Freigabe aus. Dies ermöglicht die Anwendung der Implantate bei der Immunisierung und kann in der lokalen. Tumortherapie der Toleranzentwicklung vorbeugen.

Die diskontinuierliche Freigabe eines Arzneistoffs und die sequentielle Freigabe mehrerer Arzneistoffe, wie sie zum Beispiel in der systemischen Chemotherapie oft angewendet wird, wird damit auch in der Therapie mit Implantaten möglich.

Trotz der höheren Komplexibilität der Implantate lassen sich diese mit einfachen Verfahren herstellen, die eine Massenproduktion erlauben, wie zum Beispiel Verpressen, Coaten oder Extrusion. Damit ergeben sich keine Nachteile bezüglich der Herstellung.

Die Herstellung erfindungsgemäßer Implantate kann beispielsweise wie folgt vorgenommen werden:
I. Zuerst werden die Polymere zur Herstellung der in Abbildung 1 und 2 gezeigten Arzneistoff-tragenden Schichten (Schicht 1 und 2 bzw. 1 und 4) mit Arzneistoffen beladen. Dazu eignen sich zwei Methoden.
   Die entsprechenden Polymere werden geschmolzen und die Arzneistorfe in der Schmelze gelöst oder suspendiert. Nach dem Erstarren erhält man das feste Arzneistoff-beladene Polymer. Diese Methode eignet sich zum Beispiel für alle thermostabilen Arzneistoffe.
   Die Polymere werden in einem organischen Lösungsmittel gelöst. Der Arzneistoff wird in dieser Mischung gelöst oder suspendiert. Durch Verdampfen des Lösungsmittels erhält man ein festes Polymer/Arzneistoffgemisch.
II. In einem zweiten Schritt werden die Arzneistoff-haltigen Polymere zur Herstellung rasch erodierender Schichten (Schicht 1, 2 in Abb. 1 bzw. 1 und 4 in Abb. 2) zu einem fließfähigen Pulver gemahlen.
III. Der Kern 1 des Implantates (siehe Abb. 1 und 2) wird durch Verpressen des Arzneistoff-haltigen Polymerpulvers hergestellt. Hierfür geeignet ist zum Beispiel eine hydraulische Presse oder eine Tablettenpresse.
IV. Die Herstellung eines zweischichtigen Implantats (siehe Abb. 1) erfolgt wie die Herstellung von Manteltabletten.
V. Die Arzneistoff-freien Zwischenschichten 2 und 3 für mehrschichtige Implantate (siehe Abb. 2) werden durch Verpressen von Arzneistofffreiem Polymergranulat hergestellt. Dazu wird zum Beispiel zuerst eine Bodenplatte vorgepreßt auf der der Kern 1 zentriert wird. Darüber wird eine entsprechende Menge Polymergranulat gehäuft und anschließend verpreßt. Die Aufbringung der dritten Schicht kann auch durch mehrfaches Eintauchen in eine 20-%-Polymerlösung erfolgen. Als Material wird ein bulkerodierendes Polymer verwendet. Der Körper wird anschließend über 48 Stunden getrocknet. Abschließend erfolgt ein Aufpressen eines Arzneistoff-beladenen Mantels 4 analog Schritt IV.

Die nachfolgenden Beispiele zeigen die Freigabeprofile niedermolekularer Stoffe aus einem vierschichtigen Implantat. Als Modelisubstanzen dienen Brilliantblau und Carboxyfluorescein. Die Untersuchung der Freigabe erfolgte bei 37 °C in 10 ml 0,1 M Phosphatpufferlösung pH 7,4. Die Gehaltsbestimmung erfolgte photometrisch.

Anstatt dieser Modellsubstanzen für die Freigabeuntersuchungen können beliebige Arzneistoffe eingesetzt werden.

### Beispiel 1

### Implantate mit sequentieller Freigabe zweier verschiedener niedermolekularer Stoffe:

| Verwendete Materialien: | | | |
|---|---|---|---|
| Schicht | Polymer | Farbstoff | Beladung [%] |
| Kern 1 | p(CCP-SA) 20:8* | Brilliantblau | 30 |
| Schicht 2 | p(CCP-SA) 20:8* | - | - |
| Schicht 3 | Poly(D,L-lactid) | - | - |
| Schicht 4 | p(CCP-SA) 20:8* | Carboxyfluorescein | 5 |

| | | | |
|---|---|---|---|
| *poly[1,3 bis(p-Carboxyphenoxy)Propan-co-Sebacinsäure]-20:80 | | | |

| Geometrische Abmessungen und Gewicht: | | | |
|---|---|---|---|
| Implantat | Höhe [mm] | Ø [mm] | Gewicht [mg] |
| Kern 1 | 0,95 ± 0,05 | 4 | 14,0 ± 0,5 |
| Schicht 2 | 1,74 ± 0,03 | 6 | 56,4 ± 1,1 |
| Schicht 3 | 1,90 ± 0,07 | n.b.** | 64,8 ± 1,5 |
| Schicht 4 | 3,52 ± 0,04 | 8 | 202,2 ± 2,3 |

| | | | |
|---|---|---|---|
| **nicht bestimmt | | | |

Abbildung 3 zeigt die Freigabe aus Implantaten, bei denen zuerst Carboxyfluorescein und danach Brilliantblau freigegeben wird. Die Freigabe von Brilliantblau während der ersten 10 Tage ist weitgehend unterdrückt und setzt erst nach dieser Zeit verstärkt ein.

### Beispiel 2

### Implantate mit sequentieller Freigabe desselben Stoffs

| Verwendete Materialien: | | | |
|---|---|---|---|
| Schicht | Polymer | Farbstoff | Beladung [%] |
| Kern 1 | p(CCP-SA) 20:8* | Brilliantblau | 60 |
| Schicht 2 | p(CCP-SA) 20:8* | - | - |
| Schicht 3 | Poly(D,L-lactid) | - | - |
| Schicht 4 | p(CCP-SA) 20:8* | Brilliantblau | 5 |

| | | | |
|---|---|---|---|
| *poly[1,3 bis(p-Carboxyphenoxy)Propan-co-Sebacinsäure]-20:80 | | | |

| Geometrische Abmessungen und Gewicht: | | | |
|---|---|---|---|
| Implantat | Höhe [mm] | Ø[mm] | Gewicht [mg] |
| Kern 1 | 0,89 ± 0,03 | 4 | 14,6 ± 0,5 |
| Schicht 2 | 2,55 ± 0,06 | 6 | 89,4 ± 2,8 |
| Schicht 3 | 2,92 ± 0,07 | n.b.** | 103,9 ± 3,9 |
| Schicht 4 | 4,50 ± 0,09 | 8 | 268,4 ± 2,9 |

| | | | |
|---|---|---|---|
| **nicht bestimmt | | | |

Abbildung 4 zeigt die Freigabe von Brilliantblau aus solchen Systemen. In der ersten und zweiten Phase werden jeweils 50 % Brilliantblau freigegeben.

## Patentansprüche

1. Implantat mit phasenweiser Arzneistoffabgabe, das aus einem Kern und darum konzentrisch angeordneten Schichten besteht, wobei der Kem und zumindest eine der Schichten unterschiedliche Arzneistoffe oder denselben Arzneistoff in unterschiedlicher Dosis enthalten,
**dadurch gekennzeichnet,**
**dass** die arzneistoffhaltigen Bereiche des Implantats aus einem oberflächenerodierend bioabbaubaren Polymermaterial bestehen und zwischen mindestens zwei arzneistoffhaltigen Bereichen eine oder mehrere arzneistofffreie Schichten aus bioabbaubaren Polymermaterial angeordnet sind, wobei mindestens eine der arzneistofffreien Zwischenschichten aus bulkerodierend bioabbaubaren Polymermaterialien besteht.

2. Implantat nach Anspruch 1,
**dadurch gekennzeichnet,**
**dass** die arzneistoffhaltigen Bereiche aus Polymermaterialien bestehen, welche unterschiedliche Abbaugeschwindigkeiten besitzen.

3. Implantat nach Anspruch 1 oder 2,
**dadurch gekennzeichnet,**
**dass** es zumindest aus einem Kern und einer Hüllschicht besteht, die unterschiedliche Arzneistoffe enthalten.

4. Implantat nach Anspruch 1 oder 2,
**dadurch gekennzeichnet,**
**dass** es zumindest aus einem Kern und einer Hüllschicht besteht, die denselben Arzneistoff in unterschiedlicher Dosis enthalten.

## Claims

1. Implant with phased release of pharmaceutical agents, which comprises a core and layers arranged concentrically around it, wherein the core and at least one of the layers contain different pharmaceutical agents or the same pharmaceutical agent in different doses, **characterised in that** the regions of the implant containing pharmaceutical agent are made of a surface-eroding biodegradable polymer material and one or more pharmaceutical agent-free layers of biodegradable polymer material are arranged between at least two regions containing pharmaceutical agent, wherein at least one of the pharmaceutical agent-free intermediate layers is made of bulk-eroding biodegradable polymer materials.

2. Implant according to Claim 1, **characterised in that** the regions containing pharmaceutical agent are made of polymer materials which have different rates of degradation.

3. Implant according to Claim 1 or 2, **characterised in that** it at least comprises a core and a covering layer, which contain different pharmaceutical agents.

4. Implant according to Claim 1 or 2, **characterised in that** it at least comprises a core and a covering layer, which contain the same pharmaceutical agent in different doses.

## Revendications

1. Implant avec libération de substances médicamenteuses par phases, constitué d'un noyau et de couches disposées concentriquement autour, le noyau et au moins l'une des couches contenant des substances médicamenteuses différentes ou la même substance médicamenteuse en diverses doses, **caractérisé en ce que** les zones de l'implant contenant des substances médicamenteuses sont constituées d'un matériau polymère biodégradable érodant les surfaces et **en ce qu'**une ou plusieurs couches, exemptes de substances médicamenteuses, constituées du matériau polymère biodégradable, sont disposées entre au moins deux zones contenant des substances médicamenteuses, au moins l'une des couches intermédiaires exemptes de substances médicamenteuses étant constituée de matériaux polymères biodégradables érodant la masse.

2. Implant selon la revendication 1, **caractérisé en ce que** les zones contenant des substances médicamenteuses sont constituées de matériaux polymères qui possèdent diverses vitesses de dégradation.

3. Implant selon la revendication 1 ou 2, **caractérisé en ce qu'**il se compose au moins d'un noyau et d'une couche enveloppe, qui contiennent diverses substances médicamenteuses.

4. Implant selon la revendication 1 ou 2, **caractérisé en ce qu'**il se compose au moins d'un noyau et d'une couche enveloppe, qui contiennent les mêmes substances médicamenteuses à diverses doses.
